# FASCICULE DE BREVET EUROPEEN

(11) **EP 3 262 024 B1**
(45) Date de publication et mention de la délivrance du brevet: **03.04.2019**
(21) Numéro de dépôt: 16709991.0
(22) Date de dépôt: 17.02.2016
(51) Int. Cl.: C07C 227/08, C07C 229/08, C07C 51/363, C07C 53/16, C07C 53/19, C12P 7/52, C12P 7/54

(54) **PROCÉDÉ DE PRODUCTION D'ACIDES AMINÉS À PARTIR DE PRÉCURSEURS OBTENUS PAR FERMENTATION ANAÉROBIE À PARTIR DE BIOMASSE FERMENTESCIBLE**
VERFAHREN ZUR HERSTELLUNG VON AMINOSÄUREN AUS VORLÄUFERN, DIE DURCH DIE ANAEROBE GÄRUNG VON VERGÄRBARER BIOMASSE HERGESTELLT WERDEN
PROCESS FOR PRODUCING AMINO ACIDS FROM PRECURSORS OBTAINED BY ANAEROBIC FERMENTATION FROM FERMENTABLE BIOMASS

(30) Priorité: 27.02.2015 FR 1551673
(43) Date de publication de la demande: 03.01.2018
(73) Titulaire: Afyren, 63360 Saint-Beauzire (FR)
(72) Inventeur: NOUAILLE, Régis, 63800 Cournon D'auvergne (FR); PESSIOT, Jérémy, 58400 La Charite Sur Loire (FR); THIEULIN, Marie, 10120 Saint Andre Les Vergers (FR)
(74) Mandataire: Dennemeyer & Associates S.A.
(86) Numéro de dépôt international: PCT/FR2016/050364
(87) Numéro de publication internationale: WO 2016/135397

(56) Documents cités:
- EP-A1- 0 295 550
- CN-B- 102 030 669
- US-A- 3 642 887
- US-A- 4 377 638
- HARRY H. SISLER ET AL: "STUDIES IN AMMONOLYSIS. II. AMMONOLYSIS OF [alpha]-HALOGEN ACIDS IN LIQUID AMMONIA 1", THE JOURNAL OF ORGANIC CHEMISTRY, vol. 06, no. 4, 1 juillet 1941 (1941-07-01), pages 467-478, XP055238701, US ISSN: 0022-3263, DOI: 10.1021/jo01204a001
- W. H. PERKIN ET AL: "Ueber die Einwirkung des Broms auf Essigsäure", JUSTUS LIEBIGS ANNALEN DER CHEMIE, vol. 108, no. 1, 1 janvier 1858 (1858-01-01), pages 106-113, XP055238694, WEINHEIM; DE ISSN: 0075-4617, DOI: 10.1002/jlac.18581080121

## Description

La présente invention concerne un procédé de production d'acides aminés à partir de précurseurs obtenus par fermentation anaérobie à partir de biomasse fermentescible.

Les acides aminés sont les composés constitutifs des peptides et donc des protéines. Ils sont utilisés, entres autres, comme additifs en alimentation animale (par exemple la lysine, la méthionine ou la thréonine), comme exhausteurs de gout en alimentation humaine tels que le glutamate, la sérine ou l'acide aspartique, comme nutriments spécifiques dans le domaine médical ou encore dans le domaine cosmétique. On peut également citer la glycine, l'alanine, la norvaline et la norleucine comme acides aminés ayant des applications dans le domaine de la pharmacopée, des cosmétiques et de la chimie industrielle.

On connait la production d'acides aminés par synthèse chimique ou par conversion à l'aide d'enzymes. Par exemple H.H. Sisler et al., J.Org. Chem. 6(4) 1941, p.467 - 478 et W.H. Perkin et al., Justus Liebigs Ann. der Chemie, 108(1), 1958, p.106 - 113 décrivent la préparation d'acides aminés à partir d'un acide α-halogéné. De tels procédés, bien que facilement adaptables et permettant une maitrise optimale des paramètres de production, sont complexes et onéreux à mettre en oeuvre.

Afin de minimiser les coûts de production, il existe, par exemple, des procédés d'obtention d'acides aminés par voie microbienne. Les acides aminés sont des métabolites primaires produits par des microorganismes lors d'un procédé de fermentation. Si de tels procédés permettent de produire de grandes quantités d'acides aminés directement assimilables par l'organisme, il n'en demeure pas moins que ce type de procédé est dédié à un seul type d'acides aminés.

L'invention vise plus particulièrement à remédier à ces inconvénients en proposant un procédé de production d'acides aminés permettant de produire divers types d'acides aminés, notamment non protéinogènes, de manière aisée et sans les contraintes liées aux modes de production connus de l'état de l'art.

A cet effet, l'invention a pour objet un procédé de production d'acides aminés à partir de molécules d'acides gras volatils (AGV), dites précurseurs, produites par fermentation anaérobie à partir de biomasse fermentescible, caractérisé en ce qu'il comprend au moins les étapes suivantes :
- a) extraire les molécules d'acides gras volatils (AGV), sans interruption de la fermentation, par un moyen d'extraction choisi parmi des moyens qui sont, au moins, insolubles dans le milieu de fermentation,
- b) collecter, en dehors du réacteur de fermentation, les molécules d'acides gras volatils (AGV) une fois extraites,
- c) synthétiser, par halogénation, à partir d'un type d'acide gras volatil (AGV) choisi parmi les acides gras volatils collectés à l'étape b) et défini selon le type d'acide aminé voulu, un acide α-halogéné donné,
- d) synthétiser à partir de cet acide α-halogéné un acide aminé défini.

Ainsi, un tel procédé permet de coupler une phase de production en continu de précurseurs par des microorganismes avec une phase de synthèse réalisée hors fermentation, ce qui permet un contrôle aisé des différents paramètres, tout en autorisant une plus grande variabilité dans le type d'acides aminés produits.
Un tel procédé permet de disposer, en continu, de précurseurs, à savoir des acides gras volatils, tout en préservant la capacité de production des microorganismes présents dans le bioréacteur.

En effet, les étapes a) et b) d'extraction et de collecte permettent non seulement d'extraire et de collecter en continu les molécules d'acides gras volatils produites dans le réacteur de fermentation mais également de préserver les microorganismes responsables de cette production. En effet, l'extraction, et de facto la collecte, est effectuée dans des conditions au moins non létales pour la totalité des microorganismes, c'est-à-dire dans des conditions d'extraction et de collecte biocompatibles, cela du fait que l'extraction préserve l'activité des microorganismes et que la collecte s'effectue en dehors du réacteur de fermentation.

De cette manière, on s'affranchit des problèmes liés à l'accumulation des métabolites dans le réacteur de fermentation, par exemple de l'acidification du milieu de fermentation par accumulation des acides gras volatils produits qui sont nocifs pour les microorganismes. On maintient à un niveau élevé, proche du niveau initial, la quantité et l'activité des microorganismes tout au long du cycle de fermentation.

En disposant d'une production continue et régulière d'AGV, on a une source de précurseurs variés aisément utilisable et de manière rapide. Dans le procédé objet de l'invention, cette utilisation se fait, à partir de l'étape c), par synthèse chimique et donc dans des conditions aisément contrôlables et modifiables, cela en offrant de plus une grande variabilité dans le type de molécules synthétisées. En effet, lors de l'étape c), selon l'AGV retenu pour effectuer l'halogénation, on obtient un type donné d'acide α-halogéné et donc, par la suite, un type donné de acide α-aminé.

Un tel procédé permet, lors de la phase de fermentation anaérobie d'utiliser de la biomasse fermentescible. Par biomasse fermentescible, on désigne ici un substrat organique, avantageusement non alimentaire, obtenu à partir de déchets, sous-produits et coproduits formés de matières organiques, c'est-à-dire de la biomasse, issue des activités humaines, qu'elles soient domestiques, industrielles, agricoles, forestières, aquacoles, agro-industrielles, issue de l'élevage ou autre. A titre d'exemple non limitatif, on peut citer comme substrat organique les fumiers, la fraction fermentescible des ordures ménagères, les coproduits d'abattoir, des résidus cellulosiques ou ligno-cellulosiques provenant de l'agro-industrie tels ceux issus de la transformation de la canne à sucre (bagasse), du tournesol ou du soja.

Par fermentation anaérobie on entend une fermentation réalisée dans des conditions anaérobies par des microorganismes, eucaryotes ou procaryotes, tels que des bactéries, des champignons, des algues ou des levures.

Selon des aspects avantageux mais non obligatoires de l'invention, un tel procédé peut comprendre une ou plusieurs des caractéristiques suivantes:
- Lors de l'étape c), le composé halogéné utilisé est du dibrome.
- Lors de l'étape c), le composé halogéné utilisé est différent du dibrome.
- Lors de l'étape c), on utilise de l'anhydride acétique dans un pourcentage molaire par rapport à l'acide gras volatil voisin de 12%.
- Lors de l'étape c), on utilise un anhydride correspondant à l'acide gras volatil (AGV) à halogéner.
- Lors de l'étape c), la température à laquelle la réaction de bromation est réalisée est inférieure de 20°C à 40°C à la température d'ébullition de l'acide gras volatil.
- Lors de l'étape d), la synthèse est effectuée par réaction avec de l'ammoniaque en excès par rapport à la stoechiométrie de la réaction.
- Lors de l'étape d), la synthèse est effectuée par réaction avec une amine.
- Lors de l'étape d), la température est comprise entre 20°C et 50°C.
- Lors de l'étape d), on synthétise au moins un coproduit définit comme un iminodiacide et/ou un nitrilotriacide.

Il existe plusieurs types d'acides aminés présentant un intérêt pour une utilisation industrielle, cosmétique, médicale, alimentaire ou autre. A titre d'exemple on peut citer les acides aminés non protéinogènes, tels que l'homoalanine, la norvaline, la norleucine recherchées pour la synthèse de molécules plateformes pour la pharmacopée.

On désigne ici par l'expression acide aminé des acides ayant au moins une fonction amine primaire, secondaire ou tertiaire.

Par ailleurs, grâce au procédé de l'invention, on peut réaliser la synthèse de plusieurs types d'acides aminés, tels mais non exclusivement ceux cités précédemment, de manière régulière et contrôlée, à partir d'un substrat bio-sourcé en associant une production par voie biologique avec une production par voie chimique.

L'invention sera mieux comprise et d'autres avantages de celle-ci apparaitront plus clairement à la lecture de la description de plusieurs modes de réalisation de l'invention, donnée à titre d'exemple non limitatif.

Les différentes étapes du procédé sont maintenant décrites en référence à plusieurs modes de réalisation, étant entendu que les étapes connues en soi ne sont pas détaillées.

Tout d'abord le substrat utilisé est, avantageusement, non traité, à savoir qu'il n'a subi aucun prétraitement physico-chimique ou enzymatique. Ce substrat est majoritairement constitué par de la biomasse fermentescible. A titre d'exemple complémentaire non limitatif, on peut citer les déchets agricoles ou végétaux (paille, bagasse, drèches de maïs, herbes, bois, tontes) les déchets papetiers (carton, papier), les déchets agroalimentaires, les déchets d'abattoirs, la fraction fermentescible des ordures ménagères, les effluents d'élevage (fumiers, lisiers, fientes), les algues, les déchets d'aquaculture, les déchets d'activité forestière ou encore les coproduits fermentescibles de l'industrie cosmétique. Certains substrats contiennent des molécules organiques, telles des acides organiques, qui n'influeront pas, ou de façon marginale, sur le procédé de fermentation. En revanche, ces molécules peuvent se retrouver dans le milieu de fermentation et participer, par exemple, à la production des molécules organiques finales définies.

Pour mémoire et de façon connue, le substrat est introduit dans un réacteur de fermentation, connu en soi et dimensionné pour la production souhaitée, que cette dernière soit à l'échelle du laboratoire pour effectuer des essais ou à l'échelle industrielle dans le cas d'une production. En d'autres termes, le réacteur de fermentation ou bioréacteur a un volume variant de quelques litres à plusieurs centaines de mètres cubes, selon les besoins.

Des microorganismes sont, avantageusement, introduits initialement dans le réacteur de fermentation, en quantité suffisante pour débuter la fermentation. Les microorganismes sont, avantageusement, inoculés sous forme d'un consortium. Par le terme consortium, on désigne un mélange ou mix de microorganismes, eucaryotes et procaryotes, qu'il s'agisse de bactéries, levures, champignons ou algues. Ces différents microorganismes proviennent essentiellement d'écosystèmes naturels, avantageusement mais non exclusivement, d'écosystèmes anaérobies tels que, à titre d'exemple non limitatif, la zone anaérobie des milieux aquatiques telle la zone anoxique de certains lacs, les sols, les marais, les boues d'épuration, le rumen des ruminants ou l'intestin des termites. Il convient de garder à l'esprit que la distribution qualitative et quantitative des différents types et espèces de microorganismes dans le consortium n'est pas connue précisément et surtout peut varier dans des proportions importantes. Il s'avère que cette diversité qualitative et quantitative apporte de façon surprenante une robustesse et une adaptabilité des microorganismes qui permettent d'assurer une utilisation optimale des substrats, quel que soit la composition de ces derniers et cela dans des conditions de fermentation variables.

Par ailleurs, du fait que le substrat est utilisé tel quel, c'est-à-dire qu'il n'est pas stérilisé ou, plus généralement, qu'il n'est pas débarrassé des microorganismes qu'il contient préalablement à son introduction dans le bioréacteur, il s'avère que les microorganismes endémiques au substrat sont, de facto, incorporés dans le consortium ou du moins associés à ce dernier dans le bioréacteur.

Par ailleurs, la fermentation a lieu en conditions anaérobies, plus précisément lorsque le potentiel redox est inférieur à -300mV, avantageusement compris entre -550mV et -400mV et lorsque le pH est inférieur à 8, de préférence compris entre 4 et 7. La fermentation est, avantageusement, limitée à la production de métabolites fermentaires dits précurseurs, à savoir des acides gras volatils ou AGV ayant de deux à huit carbones, préférentiellement de deux à six. On induit ainsi une réaction similaire au phénomène d'acidose rencontré chez les ruminants tout en ayant une production de méthane proche de zéro. Le méthane est, généralement, un des métabolites fermentaires finaux obtenus lors d'une fermentation anaérobie par des microorganismes issus d'écosystèmes naturels.

La fermentation conduit, dans un premier temps, à la formation d'acides gras volatils ayant, principalement, de deux à quatre carbones tels que, par exemple, l'acide acétique, l'acide propionique et l'acide butyrique. On obtient aussi, en quantité moindre, des acides gras volatils à longue chaine, donc supérieure à quatre carbones, tels que les acides valérique et caproïque, heptanoïque ou octanoïque. En poursuivant la fermentation et/ou en augmentant la quantité de microorganismes dans le bioréacteur, si besoin avec des microorganismes sélectionnés, il est possible de favoriser la production d'AGV à longue chaine carbonée, donc supérieure à quatre carbones.

En d'autres termes, les acides gras volatils produits en quantité lors de la fermentation sont essentiellement des acides gras volatils de deux à six carbones.

La fermentation est, dans tous les cas, menée pour assurer la production d'AGV, en phase liquide. Typiquement, la période de fermentation est comprise entre 1 et 7 jours, préférentiellement entre 2 et 4 jours. La concentration en métabolites obtenue dans le milieu de fermentation à l'issue de cette période est variable, mais, pour des acides gras volatils, est généralement de l'ordre de 10 à 20 g/L, selon les acides gras volatils, étant entendu que dans certaines conditions elle peut être supérieure à 35 g/L, par exemple voisine de 50 g/L. A la fin de l'étape de fermentation, le milieu de fermentation est à un pH acide, qui est généralement compris entre 4 et 6, du fait de la présence des acides gras volatils dans le milieu de fermentation.

Lorsque la production en AGV atteint une quantité définie, généralement en phase de régime permanent de la fermentation, l'étape a) d'extraction des molécules est initiée. De manière préférée mais non obligatoire, cette quantité définie d'AGV correspond à un ralentissement de la croissance des microorganismes, donc au voisinage d'un seuil d'inhibition des microorganismes.

Le moyen d'extraction est choisi parmi des moyens d'extraction, liquide ou solide, qui sont, au moins, insolubles dans le milieu de fermentation. Lorsque le moyen d'extraction est liquide, donc lorsqu'il s'agit d'un solvant, préférentiellement, la densité du solvant est inférieure à celle du milieu de fermentation.

De manière plus précise, l'extraction est conduite avec un moyen d'extraction, solide ou liquide, dont les conditions de mises en oeuvre permettent de préserver l'activité et/ou la croissance des microorganismes dans les conditions de fermentation régnant dans le bioréacteur et qui sont définies pour réaliser la fermentation. Les molécules d'AGV sont préférentiellement extraites par familles moléculaires puis avantageusement séparées individuellement par des techniques connues en soi.

Lorsque l'on extrait du milieu de fermentation des molécules telles que des acides gras volatils, de facto on réduit l'acidification du milieu de fermentation par ces acides. Ainsi, la fermentation, et donc la production de métabolites, se poursuit dans des conditions similaires aux conditions initiales, le milieu de fermentation restant peu acide.

L'extraction est, avantageusement, conduite en continu ou au moins de manière séquentielle, par exemple avec une extraction toutes les 12 heures. En d'autres termes, il est possible de poursuivre la fermentation tout en extrayant les métabolites produits, soit au fur et à mesure de leur production soit de manière régulière.

L'extraction liquide-liquide avec des solvants organiques comme moyen d'extraction est le mode d'extraction, préférentiellement mais non exclusivement, retenu.

Dans un mode de réalisation, l'extraction n'est pas réalisée dans un organe distinct du réacteur de fermentation mais directement dans ce dernier. Le solvant étant, par exemple, introduit par un dispositif de type bulleur situé en partie basse du réacteur. En variante, un organe d'extraction est couplé avec le réacteur, une communication avec le milieu de fermentation étant ménagée.

A l'issue de l'extraction, l'étape b) de collecte est mise en oeuvre. Lors de cette étape, les AGV sont collectés à partir de la phase organique par des techniques connues en soi, telles que la distillation ou l'évaporation.

La collecte est effectuée soit en mélange d'AGV soit par type d'AGV. On conçoit que le choix de l'AGV ou du mélange d'AGV est déterminé par le type de molécule(s) finale(s) souhaitée(s). Pour cela, les conditions de collecte, typiquement les paramètres d'évaporation ou de distillation, sont adaptées.

Une fois cette étape de collecte réalisée, on effectue l'étape c) suivante. Celle-ci est, avantageusement mais non exclusivement, effectuée à la suite de l'étape de collecte. En variante, elle est réalisée à un autre moment et/ou un autre endroit, les AGV produits étant transportés et/ou stockés, selon des techniques connues en soi.

Cette étape d'halogénation consiste à faire agir un halogène avec un AGV afin de produire un acide α-halogéné qui est un type de molécule très réactive et donc particulièrement intéressante pour produire d'autres molécules. Une telle réaction, connue en soi, est effectuée par ajout de brome, cela de manière préférée, étant entendu que l'on peut utiliser les autres halogènes, à savoir le chlore, le fluor ou l'iode ou des molécules halogénées telles que les trihalogénures de phosphore, les acides halogénés ou les halogénures d'acyle.

Le dibrome a été retenu car un α-haloacide bromé est plus réactif que l'a-haloacide chloré correspondant, une liaison carbone-brome étant plus facile à rompre qu'une liaison carbone-chlore. De plus, le dibrome est plus aisé à manipuler du fait de sa forme liquide.

Pour réaliser la synthèse d'acide α-bromé la voie utilisant un anhydride, ici de l'anhydride acétique, et de la pyridine a été retenue. On conçoit que d'autres voies de synthèse, par exemple avec de l'acide polyphosphorique ou les trihalogénures de phosphore, sont connues en soi. Des essais avec de l'acide polyphosphorique ont été menés mais les résultats n'ont pas été concluants, entre autre du fait de la viscosité élevée de ce composé qui rend sa manipulation difficile.

Des essais de chloration ont également été menés par la demanderesse pour la synthèse d'acides α-chlorés, par exemple avec de l'acide trichloroisocyanurique. Les résultats obtenus sont inférieurs, en termes de rendement et de facilité de mise en oeuvre à ceux obtenus avec le dibrome.

La voie de synthèse mettant en oeuvre un anhydride correspondant à l'acide gras volatil que l'on veut halogéner est intéressante et permet d'obtenir un acide α-halogéné, ici un acide α-bromé d'un type donné. L'utilisation de l'anhydride acétique avec d'autres AGV et/ou sur un mélange d'AGV de deux à six carbones permet d'obtenir un mélange d'acides α-halogénés de deux à six carbones.

Des essais mettant en oeuvre de l'acide acétique (AGV à deux carbones), de l'acide propionique (AGV à trois carbones), de l'acide butyrique (AGV à quatre carbones), de l'acide caproïque (AGV à six carbones) ainsi qu'un mélange d'AGV de deux à six carbones ont été réalisés en faisant varier la quantité d'anhydride acétique ainsi que d'autres paramètres tels que la température.

Lors des différents essais, un protocole est respecté. Il s'agit, en phase préliminaire, de chauffer à reflux un mélange initial d'AGV, d'anhydride acétique et de pyridine. Ensuite, lors de la bromation proprement dite, le dibrome est ajouté lentement, pendant plusieurs heures, à une température inférieure à la température d'ébullition du mélange, une fois le dibrome ajouté, le mélange est porté à nouveau à reflux avant d'être refroidi. En fin de réaction, avantageusement, de l'eau est ajoutée pour détruire l'anhydride présent. L'acide α-bromé est extrait ensuite par différentes méthodes, selon l'acide. Il s'agit par exemple, de distillation, d'extraction séparative.

La température initiale, pour porter le mélange à reflux, est comprise entre 120°C, pour les AGV à deux carbones et 200°C, pourles AGV à six carbones. La température de bromation varie de 80°C à 180°C, selon que les AGV ont de deux à six carbones. Le temps de la réaction de bromation, donc de facto le temps d'ajout du dibrome, varie d'environ une heure pour les AGV à six carbones à environ quatre heures pour les AGV à deux carbones.

Des essais de bromation des acides gras volatils à deux, trois, quatre, six carbones ont été effectués ainsi qu'un essai sur un mélange d'acides gras volatils:
Acide acétique (C2) : 0,53 mol
Acide propionique (C3) : 0,53 mol
Acide butyrique (C4) : 0,53 mol
Acide caproïque (C6) : 0,24 mol
Mélange d'AGV de C2 à C6 : 0,54 mol.

La quantité de dibrome ajoutée est 0,21 mol ou 0,11 mol de telle sorte que l'acide gras volatil soit en excès. Avantageusement, la demanderesse a constaté qu'un rapport molaire de 2:1 en faveur de l'AGV est optimal.

La quantité d'anhydride ajoutée est, pour chaque acide, de 0,06 mol pour un essai et de 0,03 mol pour un autre essai.

Le mélange d'AGV comprend, les acides acétique (C2), propionique (C3), butyrique (C4), valérique (C5) et caproïque (C6).

Les températures de reflux, lors de la phase préliminaire varient selon l'AGV : 120°C pour l'acide acétique ; 120°C et 140°c pour les essais avec l'acide propionique ; 150°C et 160°c pour l'acide butyrique ; 200°C pour l'acide caproïque et 180°C pour le mélange.

Les températures de bromation pour les différents essais avec chaque acide sont inférieures de 10°C à 50°C, et avantageusement de 20°C à 40°C, aux températures de reflux, donc d'ébullition de l'acide gras volatil.

Les rendements et les puretés des acides α-bromés obtenus à l'issue des différents essais sont repris ci-dessous dans le tableau 1 dans lequel les AGV sont, pour simplifier, désignés par le nombre de carbones.

**TABLEAU 1**

| Acides | Quantité anhydride (mol) | Temps de bromation (h) | Température de reflux (°C) | Température de bromation (°C) | Rendement (%) | Pureté (%) |
|---|---|---|---|---|---|---|
| C2 | 0,06 | 2,2 | 120 | 100 | 87 | 98 |
| C2 | 0,03 | 4 | 120 | 90 | 80 | 93 |
| C3 | 0,06 | 3 | 120 | 80 à 110 | 77 | 80 |
| C3 | 0,03 | 3 | 140 | 125 | 100 | 93 |
| C4 | 0,06 | 1,25 | 160 | 140 | 80 | 95 |
| C4 | 0,03 | 3 | 150 | 110 à 140 | 100 | 96 |
| C6 | 0,03 | 0,92 | 200 | 150 | 100 | NC |
| mélange | 0,06 | 1,5 | 180 | 130 | 100 | NC |

L'analyse et les calculs de rendement ont été réalisés par des techniques analytiques connues en soi, à savoir par RMN (Résonnance Magnétique Nucléaire) et par HPLC (High Performance Liquid Chromatography). Les rendements sont définis par rapport à la quantité d'AGV consommée.

La demanderesse a constaté que la vitesse de la réaction, illustrée par la décoloration du mélange réactionnel après ajout du dibrome, est plus rapide lorsque la quantité d'anhydride est plus importante, la pureté étant peu affectée. Néanmoins, il convient que la température des deux étapes, préliminaire et de bromation, soit optimale.

Pour cela, la demanderesse a noté qu'une température de bromation inférieure à la température d'ébullition de l'acide gras volatil est nécessaire, cela sans être trop éloignée de cette température.

Les différents essais ont permis de définir qu'une température de bromation inférieure d'environ 10°C à 50°C à la température d'ébullition de l'acide gras volatil et, avantageusement, inférieure de 20 °C permettait, toute autre condition étant identique, d'obtenir un rendement optimal, typiquement entre 60% et 100% avec un temps de réaction de 1h à 4h.

Concernant le rôle de l'anhydride acétique, à la vue des résultats du tableau, il apparait que le pourcentage molaire d'anhydride par rapport à l'AGV doit être voisin de 12% pour une réaction de bromation optimale, étant entendu qu'un pourcentage compris entre 5% et 20% est acceptable.

A partir des acides α-bromés obtenus, ou plus précisément à partir d'un acide α-bromé donné, on réalise ensuite la synthèse, lors de l'étape d), d'un acide α-aminé donné. Pour cela on ajoute de l'ammoniaque, sous forme gazeuse ou en solution. En variante, l'ammoniaque est remplacée par une amine primaire ou secondaire.

Des essais ont été effectués par la demanderesse par réaction de l'ammoniaque sur des α-bromoacides ayant de deux à six carbones, à savoir sur l'acide bromoacétique, l'acide α-bromopropionique, l'acide α-bromobutyrique, l'acide α-bromovalérique, l'acide α-bromocaproïque. Une telle réaction permet l'obtention d'acides α-aminés ayant une chaine carbonée respectivement de deux, trois, quatre, cinq ou six carbones, soit la glycine, l'alanine, l'homoalanine, la norvaline et la norleucine.

Ces acides α-aminés sont parmi les plus utilisés comme constitutifs de produits cosmétiques, alimentaires, que ce soit en alimentation humaine ou animale ou comme intermédiaires réactionnels dans la chimie et la pharmacopée. On conçoit aisément que le procédé objet de l'invention permet la production d'autres types d'acides aminés.

Pour les différents essais, le protocole a consisté à faire agir, à une température comprise entre la température ambiante, soit environ 20°C, et 50°C, de l'ammoniaque avec l'acide α-bromé. La réaction est menée pendant un temps variable, allant d'une demi-heure à soixante-douze heures selon le type d'acide α-bromé et selon la température. Un acide α-bromé long, c'est-à-dire ayant au moins quatre carbones, nécessite un temps de réaction long, typiquement supérieur à 24 heures à température ambiante mais inférieur à 12h à 50°C.

La demanderesse a constaté, de façon surprenante qu'une température de réaction élevée et voisine de 50°C permet de diminuer le temps de réaction.

La demanderesse a constaté que lorsque l'ammoniaque est mise en excès, à savoir dans un rapport molaire voisin de 1:10, la conversion en acide aminé est optimisée.

Le tableau 2 suivant reprend les résultats obtenus.

L'analyse et les calculs de rendements et des taux de conversion ont été réalisés par les techniques analytiques de RMN (Résonnance Magnétique Nucléaire) et HPLC (High Performance Liquid Chromatography). Les taux de conversion sont calculés à partir de la quantité en acide aminé produite dans le milieu réactionnel par rapport à la quantité initiale en acide α-bromé. Les rendements sont définis par rapport à l'analyse massique et qualitative des produits, récupérés après extraction et recristallisation dans le méthanol.

**TABLEAU 2**

| Acides | Ratio acide :NH3 | T (°C) | Consommation en acide α-bromé(%) - durée (h) | Conversion en AA (%) | Rendement après extraction (%) |
|---|---|---|---|---|---|
| C2 | 1 :10 | (T ambiante) | 99% - 5h | 46 | 24 |
| C3 | 1 :5 | (T ambiante) | 95% - 5h | 65 | 44 |
| C3 | 1 :10 | (T ambiante) | 98%- 5h | 66 | 52 |
| C3 | 1 :10 | 50 | 99% - 1h | 64 | 40 |
| C4 | 1 :5 | (T ambiante) | 96% - 24h | 55 | 39 |
| C4 | 1 :10 | (T ambiante) | 99% - 24h | 76 | 54 |
| C4 | 1 :10 | 50 | 99% - 2h | 58 | 48 |
| C6 | NH3 en excès | (T ambiante) | 99% - 72h | / | 43 |

On obtient ainsi une production d'acides aminés à partir d'une source bio-sourcée, cela avec des conditions de production aisées à mettre en oeuvre et à contrôler.

En complément, les taux de conversion en acide aminé étant inférieurs aux taux de consommation en acide α-bromé, il en ressort la synthèse d'au moins un co-produit définit comme des iminodiacides et/ou nitrilotriacides. A titre d'exemple non exhaustif, pour la synthèse de la glycine, les co-produits sont, entre autres, l'acide iminodiacétique et l'acide nitrilotriacétique et pour la synthèse de l'alanine, les co-produits sont, entre autres, l'acide α,α'- iminodipropionique et l'acide α,α', α"-nitrilotripropionique.

## Revendications

1. Procédé de production d'acides aminés à partir de molécules d'acides gras volatils (AGV), dites précurseurs, produites par fermentation anaérobie à partir de biomasse fermentescible, **caractérisé en ce qu'**il comprend au moins les étapes suivantes :
- a) extraire les molécules d'acides gras volatils (AGV), sans interruption de la fermentation, par un moyen d'extraction choisi parmi des moyens qui sont, au moins, insolubles dans le milieu de fermentation,
- b) collecter, en dehors du réacteur de fermentation, les molécules d'acides gras volatils (AGV) une fois extraites,
- c) synthétiser, par halogénation, à partir d'un type d'acide gras volatil (AGV) choisi parmi les acides gras volatils collectés à l'étape b) et défini selon le type d'acide aminé voulu, un acide α-halogéné donné,
- d) synthétiser à partir de cet acide α-halogéné un acide aminé défini.

2. Procédé selon la revendication 1, **caractérisé en ce que**, lors de l'étape c), le composé halogéné utilisé est du dibrome.

3. Procédé selon la revendication 1, **caractérisé en ce que**, lors de l'étape c), le composé halogéné utilisé est différent du dibrome.

4. Procédé selon la revendication 1 ou 2, **caractérisé en ce que** lors de l'étape c), on utilise de l'anhydride acétique dans un pourcentage molaire par rapport à l'acide gras volatil voisin de 12%.

5. Procédé selon la revendication 1 ou 2, **caractérisé en ce que** lors de l'étape c), on utilise un anhydride correspondant à l'acide gras volatil (AGV) à halogéner

6. Procédé selon une des revendications précédentes, **caractérisé en ce que** lors de l'étape c), la température à laquelle la réaction de bromation est réalisée est inférieure de 20°C à 40°C à la température d'ébullition de l'acide gras volatil.

7. Procédé selon une des revendications précédentes, **caractérisé en ce que**, lors de l'étape d), la synthèse est effectuée par réaction avec de l'ammoniaque en excès par rapport à la stoechiométrie de la réaction.

8. Procédé selon l'une des revendications 1 à 6, **caractérisé en ce que**, lors de l'étape d), la synthèse est effectuée par réaction avec une amine.

9. Procédé selon la revendication 7 ou 8, **caractérisé en ce que**, lors de l'étape d), la température est comprise entre 20°C et 50°C.

10. Procédé selon l'une des revendications précédentes, **caractérisé en ce que** lors de l'étape d), on synthétise au moins un coproduit définit comme un iminodiacide et/ou un nitrilotriacide.

## Patentansprüche

1. Verfahren zur Herstellung von Aminosäuren aus als Vorläufer bezeichneten flüchtigen Fettsäuremolekülen (VFA), die durch anaerobe Gärung aus vergärbarer Biomasse hergestellt werden, **dadurch gekennzeichnet, dass** es mindestens die folgenden Schritte umfasst:
- a) Extrahieren der flüchtigen Fettsäuremoleküle (VFA), ohne die Gärung zu unterbrechen, durch ein Extraktionsmittel, das aus Mitteln ausgewählt ist, die mindestens in der Gärungsumgebung unlöslich sind,
- b) Auffangen der einmal extrahierten flüchtigen Fettsäuremoleküle (VFA) außerhalb des Fermentationsreaktors,
- c) Synthetisieren einer bestimmten α-halogenierten Säure durch Halogenierung aus einer Art von flüchtiger Fettsäure (VFA), die aus den in Schritt b) aufgefangenen flüchtigen Fettsäuren ausgewählt ist und nach Art der gewünschten Aminosäure definiert ist,
- d) Synthetisieren, aus dieser α-halogenierten Säure, einer definierten Aminosäure.

2. Verfahren nach Anspruch 1, **dadurch gekennzeichnet, dass** bei Schritt c) die verwendete halogenierte Verbindung Dibrom ist.

3. Verfahren nach Anspruch 1, **dadurch gekennzeichnet, dass** sich bei Schritt c) die verwendete halogenierte Verbindung von Dibrom unterscheidet.

4. Verfahren nach Anspruch 1 oder 2, **dadurch gekennzeichnet, dass** bei Schritt c) Essigsäureanhydrid in einem molaren Prozentsatz im Verhältnis zur flüchtigen Fettsäure von etwa 12 % verwendet wird.

5. Verfahren nach Anspruch 1 oder 2, **dadurch gekennzeichnet, dass** bei Schritt c) ein der zu halogenierenden flüchtigen Fettsäure (VFA) entsprechendes Anhydrid verwendet wird.

6. Verfahren nach einem der vorhergehenden Ansprüche, **dadurch gekennzeichnet, dass** bei Schritt c) die Temperatur, bei der die Bromierungsreaktion durchgeführt wird, 20 °C bis 40 °C unter der Siedetemperatur der flüchtigen Fettsäure liegt.

7. Verfahren nach einem der vorhergehenden Ansprüche, **dadurch gekennzeichnet, dass** bei Schritt d) die Synthese durch Reaktion mit Ammoniak im Überschuss, bezogen auf die Stöchiometrie der Reaktion, erfolgt.

8. Verfahren nach einem der Ansprüche 1 bis 6, **dadurch gekennzeichnet, dass** bei Schritt d) die Synthese durch Reaktion mit einem Amin erfolgt.

9. Verfahren nach Anspruch 7 oder 8, **dadurch gekennzeichnet, dass** bei Schritt d) die Temperatur im Bereich zwischen 20 °C und 50 °C liegt.

10. Verfahren nach einem der vorhergehenden Ansprüche, **dadurch gekennzeichnet, dass** bei Schritt d) mindestens ein Beiprodukt synthetisiert wird, das als eine Iminodisäure und/oder eine Nitrilotrisäure definiert ist.

## Claims

1. A process for producing amino acids from volatile fatty acid (VFA) molecules, termed precursors, produced by anaerobic fermentation from fermentable biomass, **characterised in that** said process comprises at least the following steps:
- a) extracting the volatile fatty acid (VFA) molecules without interrupting the fermentation using an extraction means chosen from among means which are, at least, insoluble in the fermentation medium,
- b) collecting, outside the fermentation reactor, the volatile fatty acid (VFA) molecules once extracted,
- c) synthesising a given α□haloacid by halogenation starting from a type of volatile fatty acid (VFA) chosen from among the volatile fatty acids collected in step b) and defined according to the type of amino acid desired,
- d) synthesising a predefined amino acid from said α□haloacid.

2. The process according to claim 1, **characterised in that** the halogen compound used during step c) is dibromine.

3. The process according to claim 1, **characterised in that** the halogen compound used during step c) is different from dibromine.

4. The process according to claim 1 or 2, **characterised in that**, during step c), acetic anhydride is used in a molar percentage close to 12% with respect to the volatile fatty acid.

5. The process according to claim 1 or 2, **characterised in that**, during step c), an anhydride corresponding to the volatile fatty acid (VFA) to be halogenated is used.

6. The process according to one of the preceding claims, **characterised in that**, during step c), the temperature at which the bromination reaction is carried out is 20°C to 40°C lower than the boiling temperature of the volatile fatty acid.

7. The process according to one of the preceding claims, **characterised in that**, during step d), the synthesis is carried out by reaction with excess ammonia relative to the stoichiometry of the reaction.

8. The process according to one of the claims 1 to 6, **characterised in that**, during step d), the synthesis is carried out by reaction with an amine.

9. The process according to claim 7 or 8, **characterised in that**, during step d), the temperature is between 20°C and 50°C.

10. The process according to one of the preceding claims, **characterised in that**, during step d), at least one co-product, defined as an iminodiacid and/or a nitrilotriacid, is synthesised.
